# EUROPÄISCHE PATENTANMELDUNG

(11) **EP 1 938 782 A1**
(43) Veröffentlichungstag der Anmeldung: **02.07.2008**
(21) Anmeldenummer: 07023932.2
(22) Anmeldetag: 11.12.2007
(51) Int. Cl.: A61K 8/02, A61Q 1/14, A61F 13/38

(54) **Abschminkmittel**

(30) Priorität: 28.12.2006 DE 102006062497
(71) Anmelder: Henkel AG & Co. KGaA, 40589 Düsseldorf (DE)
(72) Erfinder: Neubueser, Inge, 22587 Hamburg (DE)

(57) **Zusammenfassung**

Gegenstand der vorliegenden Anmeldung sind Mittel zur kosmetischen Reinigung, insbesondere zum Abschminken, umfassend einen stäbchenförmigen Einweg-Applikator, dessen Applikatorkopf mit einer Zusammensetzung zur Reinigung, insbesondere zum Abschminken, beaufschlagt ist.

## Beschreibung

Die vorliegende Erfindung betrifft ein Mittel zum Abschminken, umfassend eine kosmetische Reinigungszusammensetzung, die insbesondere zum Abschminken geeignet ist, sowie einen Einweg-Applikator hierfür.

### Stand der Technik

Das Schminken des Gesichts, der Lippen und/oder der Augen gehört für viele Verbraucher zur täglichen Körperpflege. Durch die nicht-permanente Anfärbung der Haut oder der Schleimhäute sollen Hautunebenheiten optisch ausgeglichen werden (Make-up, Foundation) oder bestimmte Partien des Gesichts farblich besonders hervorgehoben werden (Lippenstift, Augen).

Ein bekanntes Problem von färbenden Schminkzusammensetzungen, wie Lippenstift, Mascara, Kajal oder Lidschatten, besteht darin, dass sie nicht dauerhaft (das heißt, bis zur nächsten Reinigung) auf der ursprünglichen Hautpartie verbleiben, sondern im Laufe der Tragzeit - durch Transpiration, Augenflüssigkeit und Gesichtsmimik - migrieren. Dies führt zum Verschmieren oder Verlaufen des Make-ups, was den ästhetischen Eindruck stark beeinträchtigt. Üblicherweise tritt dies erst im Laufe des Tages (oder Abends) auf, beispielsweise am Arbeitsplatz, unterwegs oder während einer Abendveranstaltung. Häufig steht dem Verbraucher/der Verbraucherin dann nicht die gewohnte Reinigungsausstattung zur Verfügung bzw. es fehlt die Zeit zur ausführlichen Reinigung und Erneuerung des Make-ups.

Zusammensetzungen zum Abschminken stellen üblicherweise Gesichtswässer (Tonics), Lotionen, Gele und (insbesondere für Theaterschminken oder Camouflage-Make-up) Cremes mit höherem Fettanteil dar, die entweder direkt oder mit einem Schwamm, Pad oder Tuch auf die Haut und/oder die Schleimhaut aufgetragen und anschließend abgewischt und/oder abgespült werden.

Nachteilig bei dieser Art der Reinigung ist, dass so immer größere Hautpartien mit der Reinigungszusammensetzung in Berührung kommen. Das gezielte Reinigen und/oder Korrigieren von kleineren migrierten Schminkmengen oder Schminkfehlern, ohne gleich das gesamte Make-up erneuern zu müssen, ist so nicht möglich.

Ein weiterer Nachteil des Standes der Technik ist, dass immer eine Reinigungszusammensetzung und ein Applikator mitgeführt werden müssen, was bei den handelsüblichen Packungsgrößen für den Verbraucher unterwegs nur sehr umständlich handhabbar ist.

Daher bestand seit langem das Bestreben, geeignete Applikationsformen für Abschminkzusammensetzungen zu finden, die es ermöglichen, die Abschminkzusammensetzung möglichst gezielt auf die zu reinigenden Stellen aufzubringen, um so eine bequeme, Zeit und Material sparende Art der Reinigung bereitzustellen.

### Aufgabenstellung

Eine Aufgabe der vorliegenden Erfindung war es, ein Mittel zur kosmetischen Reinigung bereitzustellen, dass eine gezielte, möglichst kleinflächige Reinigung, beispielsweise zur Korrektur von Schminkfehlern, erlaubt.
Eine weitere Aufgabe der vorliegenden Erfindung war es, ein Mittel zur kosmetischen Reinigung bereitzustellen, dass eine praktische, platz- und zeitsparende Reinigung, insbesondere unterwegs, erlaubt.

Es wurde nun überraschend ein Mittel zum Abschminken gefunden, das die gestellten Aufgaben löst, die Nachteile des Standes der Technik vermeidet und die oben genannten Anforderungen in hohem Maße erfüllt.

Gegenstand der vorliegenden Erfindung ist daher ein Mittel zur kosmetischen Reinigung, insbesondere zum Abschminken, umfassend einen stäbchenförmigen Einweg-Applikator, dessen Applikatorkopf mit einer Zusammensetzung zur Reinigung, insbesondere zum Abschminken, beaufschlagt ist.

Ein weiterer Gegenstand der vorliegenden Erfindung ist ein nicht-therapeutisches Verfahren zur Reinigung und/oder zum Abschminken der Haut und/oder der Schleimhaut, wobei die zu reinigende Hautpartie mit einem stäbchenförmigen Einweg-Applikator, dessen Applikatorkopf mit einer Zusammensetzung zur Reinigung, insbesondere zum Abschminken, beaufschlagt ist, gereinigt wird.

Ein weiterer Gegenstand der vorliegenden Erfindung ist die Verwendung eines Mittels, umfassend einen stäbchenförmigen Einweg-Applikator, dessen Applikatorkopf mit einer Zusammensetzung zur Reinigung, insbesondere zum Abschminken, beaufschlagt ist, zur Reinigung und/oder zum Abschminken der Haut und/oder der Schleimhaut.

In einer bevorzugten Ausführungsform der Erfindung liegt die Reinigungszusammensetzung in Form einer flüssigen oder fließfähigen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, in wasserfreier Form, wie beispielsweise einem Öl oder einem Balsam, vor.

In einer weiteren bevorzugten Ausführungsform der Erfindung weist die Reinigungszusammensetzung einen Wassergehalt von 45 bis 65 Gew.-% und eine Viskosität [mPas] im Bereich von 8.000 bis 45.000 auf (gemessen mit Brookfield RVF; Spindel 4/4UpM).

In einer weiteren bevorzugten Ausführungsform der Erfindung weist die Reinigungszusammensetzung einen Wassergehalt von 5 bis 25 und eine Viskosität [mPas] im Bereich von 8.000 bis 45.000 auf (gemessen mit Brookfield RVF; Spindel 4/4UpM).

Die erfindungsgemäß applizierten Reinigungszusammensetzungen enthalten in einer bevorzugten Ausführungsform zusätzlich mindestens ein weiteres Binde- oder Verdickungsmittel. Diese wirken konsistenzregulierend und verhindern weiterhin die Separation der flüssigen und festen Bestandteile.
Ihre Einsatzmengen in den erfindungsgemäß applizierten Zusammensetzungen betragen bevorzugt 0,1 - 5 Gew.-%, besonders bevorzugt 0,1 - 3 Gew.-% und außerordentlich bevorzugt 0,5 - 2 Gew.-%, bezogen auf das Gewicht der Reinigungszusammensetzung.

Erfindungsgemäß bevorzugte Binde- oder Verdickungsmittel sind beispielsweise natürliche und/ oder synthetische wasserlösliche Polymere wie Alginate (Polymannuronat und Polyguluronat, beispielsweise Natriumalginat, Propylenglykolalginat, Calciumalginat oder Glycerylalginat), Carrageenane, Agar-Agar, Guar-Gum, Gummi arabicum, Succinoglycan-Gum, Guarmehl, Johannisbrotkernmehl, Tragant, Karaya-Gummi, Xanthan, Pektine, Scleroglucane, Chitin- oder Chitosanderivate, Cellulose und deren ionische und nicht-ionische Derivate wie beispielsweise Carboxymethylcellulose, Hydroxyethylcellulose oder Methylhydroxypropylcellulose, hydrophob modifizierte Cellulosen, beispielsweise Cetylhydroxycellulose, Stärke- und Stärkeether, heterogene Polyholoside, insbesondere solche, die zumindest eine Fucoseeinheit, Rhamnoseeinheit, Galactoseeinheit und/oder Galacturonsäureeinheit enthalten und beispielsweise unter den Handelsnamen Fucogel 1000 (INCl: Biosaccharide Gum-1) von Solabia, Rhamnosoft^{®} (INCl-Bezeichnung Biosaccharide Gum-2) von Solabia, Fucogenol^{®} (INCl-Bezeichnung Biosaccharide Gum-3) von Solabia und Glycofilm^{®} (INCl-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia, Polycarboxyvinyl-Derivate vom Carbomer-Typ (von Noveon unter den Bezeichnungen Carbopol 940, 951, 980 oder von 3V-SIGMA unter den Bezeichnungen Synthalen K oder Synthalen L im Handel erhältlich), hydrophob modifizierte Polycarboxyvinyl-Derivate, bevorzugt solche mit der INCI-Bezeichnung ACRYLATES/C10-30 ALKYL ACRYLATE CROSSPOLY-MER (von Noveon unter den Bezeichnungen Carbopol 1342, Carbopol 1382, Carbopol Ultrez 21, Carbopol ETD 2020, Pemulen TR 1 oder Pemulen TR 2 im Handel erhältlich), ein Acrylamid/ Methylpropansulfonsäure-Copolymer (Sepigel von SEPPIC), ein Natriumacrylat/Methylpropansulfonsäure-Copolymer (Simulgel EG, Simulgel EPG von SEPPIC); ein Hydroxyethylacrylat/Methylpropansulfonsäure-Copolymer (Simulgel NS von Seppic) oder ein Vinylpyrrolidon/Methylpropansulfonsäure-Copolymer Aristoflex AVC von Clariant).
Auch Polyvinylalkohol, Polyvinylpyrrolidon und höhermolekulare Polyethylenglycole (insbesondere solche mit Molekulargewichten von 10² - 10⁶ D) eignen sich als bevorzugte Binde- oder Verdickungsmittel. Ebenso können Schichtsilikate und feinteilige Kieselsäuren (Aerogelkieselsäuren und pyrogene Kieselsäuren) diese Funktion erfüllen.

Die erfindungsgemäß applizierten Reinigungszusammensetzungen enthalten in einer weiteren bevorzugten Ausführungsform zusätzlich mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon. Bevorzugt sind diese Polyol-Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit, Xylitol sowie Mischungen der vorgenannten Substanzen. Bevorzugte wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fructose, Glucose, Maltose, Maltitol, Mannit, Inosit, Sucrose, Trehalose und Xylose sind erfindungsgemäß geeignet.
Bevorzugt ist die Kombination mehrerer Polyole, wobei die Kombination von Glycerin, 1,2-Propylenglycol und/oder PEG-8 als besonders bevorzugt anzusehen ist.
Die Polyol-Komponenten sind in der Reinigungszusammensetzung bevorzugt in einer Menge von 1-20 Gew.-%, vorzugsweise 2-15 Gew.-% und insbesondere 5-10 Gew.-%, enthalten, jeweils bezogen auf das Gewicht der erfindungsgemäß applizierten Reinigungszusammensetzung.

### Öle

Es ist bekannt, abschminkende Öle in Zusammensetzungen zum Abschminken der Haut und/oder der Augen zu verwenden. Diese Zusammensetzungen enthalten im Allgemeinen außerdem grenzflächenaktive Stoffe, durch die das Öl und/oder Reste der Schminke besser entfernt werden können.
Prinzipiell sind alle bekannten kosmetischen Öle, die keine Duftstoffe darstellen, zur Verwendung in den erfindungsgemäß applizierten Reinigungszusammensetzungen geeignet, insbesondere tierische Öle, pflanzliche Öle, wie Erdnussöl, Süßmandelöl, Calophyllumöl, Palmöl, Ricinusöl, Avocadoöl, Jojobaöl, Olivenöl und Getreidekeimöl, Mineralöle, wie Paraffinöl oder Vaselineöl, Squalen, Squalan (= Perhydrosqualen), unter Normalbedingungen (1013 mbar, 20 °C) flüssige Alkohole wie Oleylalkohol, Linoleylalkohol, Linolenylalkohol, Isostearylalkohol oder Octyldodecanol, Siliconöle, wie z. B. Polydimethylsiloxane, die gegebenenfalls phenylgruppenhaltig sind, beispielsweise Phenyltrimethicone, und/oder fluorierte Öle enthalten.
Es ist aber bekannt, dass bestimmte Öle, insbesondere die pflanzlichen Öle und die Triglyceridöle, ein unangenehm fettiges, beschwerendes Gefühl hervorrufen können, insbesondere auf den Augenlidern und/oder an den Augen. Auch mineralische Öle und Dialkylether sind weniger bevorzugt, die diese gerade im Augenbereich reizend wirken können. Bevorzugte Öle sind ausgewählt aus Fettsäureestern, wobei der Fettsäureester aus einem geradkettigen oder verzweigten Fettalkohol mit 1 bis 17 Kohlenstoffatomen und einer geradkettigen oder verzweigten Fettsäure mit 3 bis 18 Kohlenstoffatomen gebildet wird. Besonders bevorzugte derartige Fettsäureester sind Isopropylmyristat, Isopropylpalmitat, Dioctyladipat, 2-Ethylhexylpalmitat, Diisopropyladipat, 2-Ethylhexylhexanoat, Ethyllaurat, Methylmyristat, Octyldodecyloctanoat, Isodecylneopentanoat, Ethylmyristat, Myristylpropionat, 2-Ethylhexyl-2-ethylhexanoat, 2-Ethylhexyloctanoat, 2-Ethylhexylcaprat/caprylat, Methylpalmitat, Butylmyristat, Isobutylmyristat, Ethylpalmitat, Isohexyllaurat, Hexyllaurat und Isopropylisostearat. Erfindungsgemäß außerordentlich bevorzugte Ölkomponenten sind ausgewählt aus Fettsäure- und Fettalkoholestern. Besonders bevorzugt sind die Ester aus einem einwertigen geradkettigen oder verzweigten Alkohol mit 1 bis 17 Kohlenstoffatomen und einer geradkettigen oder verzweigten Fettsäure mit 3 bis 24 Kohlenstoffatomen. Außerordentlich bevorzugt sind die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Methanol, Ethanol, n-Propanol, Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linoleylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol^{®} SN), Stearinsäure-2-ethylhexylester (Cetiol^{®} 868), Cetyloleat und n-Butylstearat.
Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglycol-di-isotridecanoat, Propylenglycol-di(2-ethylhexanoat), Propylenglycol-di-isostearat, Propylenglycol-di-pelargonat, Butandiol-di-isostearat und Neopentylglykoldi-capylat erfindungsgemäß bevorzugte Ölkomponenten dar, ebenso komplexe Ester wie z. B. das Diacetyl-glycerinmonostearat.

Weitere für die erfindungsgemäßen Mittel bevorzugte Öle sind ausgewählt aus Siliconölen. Erfindungsgemäß bevorzugte Siliconöle sind ausgewählt aus flüchtigen Siliconölen, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind, oder linear, z. B. Hexamethyldisiloxan (L₂), Octamethyltrisiloxan (L₃), Decamethyltetrasiloxan (L₄), beliebige Zweier- und Dreiermischungen aus L₂, L₃ und/oder L₄, wie sie z. B. in den Handelsprodukten DC 2-1184, Dow Corning^{®} 200 (0, 65 cSt) und Dow Corning^{®} 200 (1,5 cSt) von Dow Corning enthalten sind, weiterhin aus nichtflüchtigen höhermolekularen linearen Dimethylpolysiloxanen, im Handel erhältlich z. B. unter der Bezeichnung Dow Corning^{®} 190, Baysilon^{®} 350 M und diverser Produkte aus der Serie Dow Corning^{®} 200 Fluid mit kinematischen Viskositäten im Bereich von 5 - 50000 cSt, bevorzugt 5 - 10000 cSt, besonders bevorzugt 10 - 350 cSt und außerordentlich bevorzugt 50 - 100 cSt, gemessen bei 25 °C. Ganz besonders bevorzugt sind Silikonöle mit kinematischen Viskositäten, bis zu 10.000 cSt gemessen bei 25°C. Die Bestimmung der Viskositäten erfolgt dabei nach der Kugelfallmethode entsprechend der Methode "British Standard 188". Vergleichbare Werte werden mit zum "British Standard 188" analogen Prüfvorschriften der Hersteller erhalten, beispielsweise der "CTM 0577" der Dow Corning Corporation.
Eine bevorzugte Ausführungsform der Erfindung ist dadurch gekennzeichnet, dass das mindestens eine flüssige Siliconöl ausgewählt ist aus flüchtigen cyclischen Siliconölen, insbesondere Octamethylcyclotetrasiloxan, Decamethylcyclopentasiloxan und Dodecamethylcyclohexasiloxan sowie Mischungen hiervon, linearen flüchtigen Siliconölen, z. B. Hexamethyldisiloxan (L₂), Octamethyltrisiloxan (L₃), Decamethyltetrasiloxan (L₄), beliebigen Zweier- und Dreiermischungen aus L₂, L₃ und/oder L₄, weiterhin aus nichtflüchtigen höhermolekularen linearen Dimethylpolysiloxanen mit Viskositäten im Bereich von 5 - 50000 cSt.
Eine weitere bevorzugte Ausführungsform der Erfindung ist dadurch gekennzeichnet, dass das mindestens eine flüssige Siliconöl in einer Gesamtmenge von 1 - 40 Gew.-%, bevorzugt 5 - 30 Gew.-%, besonders bevorzugt 10 - 25 Gew.-%, außerordentlich bevorzugt 15 - 20 Gew.-%, jeweils bezogen auf die gesamte erfindungsgemäß applizierte Reinigungszusammensetzung, enthalten ist.
Besonders bevorzugt sind Mischungen aus den Fettsäureestern aus einem geradkettigen oder verzweigten Fettalkohol mit 1 bis 17 Kohlenstoffatomen und einer geradkettigen oder verzweigten Fettsäure mit 3 bis 18 Kohlenstoffatomen und den Siliconölen.
Eine erfindungsgemäß ebenfalls geeignete Gruppe von Ölkomponenten sind flüssige Paraffinöle und synthetische Kohlenwasserstoffe sowie Di-n-alkylether mit insgesamt 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
Die Ölphase kann im Falle einer Emulsion in einem Anteil von 5 bis 95 Gew.-% enthalten sein, bezogen auf das Gewicht der erfindungsgemäß applizierten Reinigungszusammensetzung.

### Emulgatoren

Ferner sind Zusammensetzungen zum Abschminken bekannt, die auf grenzflächenaktiven Stoffen basieren, mit denen Schminkereste entfernt werden können und die gleichzeitig das Emulgieren der Zusammensetzung erleichtern, indem sie die Fettphase und die wässrige Phase der Reinigungszusammensetzung kompatibel machen, wenn diese in Form einer Emulsion vorliegt.
Die erfindungsgemäß applizierten Zusammensetzungen können in einer bevorzugten Ausführungsform oberflächenaktive Substanzen enthalten, die, je nach Anwendungsgebiet als Tenside oder als Emulgatoren bezeichnet, aus anionischen, kationischen, zwitterionischen, ampholytischen und nichtionischen Tensiden und Emulgatoren ausgewählt sind.
Als anionische Tenside und Emulgatoren eignen sich für die erfindungsgemäßen Reinigungszusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside und Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Sulfonate ungesättigter Fettsäuren mit 8 bis 24 C-Atomen und 1 bis 6 Doppelbindungen,
- Acylglutamate der Formel (I), 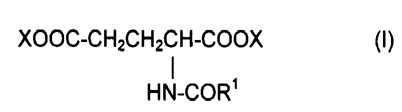 in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (II), 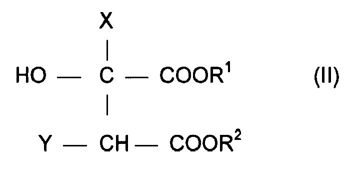 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Ester der Sulfobernsteinsäure oder der Sulfosuccinate der allgemeinen Formel (III), 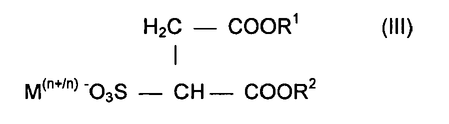 in der M^{(n+/n)} für n = 1 ein Wasserstoffatom, ein Alkalimetallkation, eine Ammoniumgruppe oder das Kation einer ammonium-organischen Base und für n = 2 ein Erdalkalimetallkation darstellt und R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel R-(O-CH₂-CH₂)ₓ-OSO₃H, in der R eine bevorzugt lineare Alkylgruppe mit 8 bis 30 C-Atomen und x = 0 oder 1 - 12 ist,
- gemischte oberflächenaktive Hydroxysulfonate gemäß DE-A-37 25 030,
- Alkyl- und/oder Alkenyletherphosphate der Formel (E1-I),

   R¹(OCH₂CH₂)ₙO-P(O)(OX)-OR² (E1-I)

   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht,
- Monoglyceridsulfate und Monoglyceridethersulfate der Formel (E1-III) 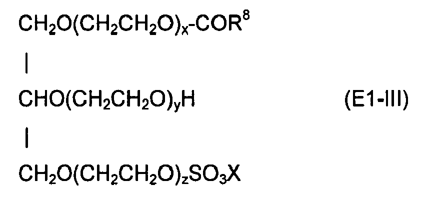 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/ oder Aminosäuren und deren Derivaten,welche dem Fachmann als Eiweißfettsäurekondensate bekannt sind, wie beispielsweise die Lamepon^{®}-Typen, Gluadin^{®}-Typen, Hostapon^{®} KCG oder die Amisoft^{®} - Typen.

Bevorzugte anionische Tenside und Emulgatoren, besonders für milde (Gesichts-)Hautreinigungs-mittel, sind Acylglutamate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Citronensäure oder Bernsteinsäure bzw. der Salze dieser Säuren mit alkylierter Glucose, insbesondere die Produkte mit der INCl-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.
Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylenester mit 8 bis 18 C-Atomen in der Alkylgruppe und 1 bis 6 Oxyethylengruppen, Monoglyceridsulfate, Alkyl- und Alkenyletherphosphate sowie Eiweißfettsäurekondensate.

Als zwitterionische Tenside und Emulgatoren werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammonium-glycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCl-Bezeichnung Cocamidopropyl Betaine bekannte Fettsäureamidderivat.

Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside (E4) und Emulgatoren enthalten als hydrophile Gruppe z. B. eine Polyolgruppe, eine Polyalkylenglycolethergruppe oder eine Kombination aus Polyol- und Polyglycolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Handelsnamen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Polyole mit 3 bis 6 Kohlenstoffatomen, insbesondere an Glycerin,
- Anlagerungsprodukte von 5 bis 60 Mol Ethylenoxid an Rizinusöl und gehärtetes Rizinusöl,
- Polyolfettsäure(partial)ester, wie Hydagen^{®} HSP (Cognis) oder Sovermol^{®}-Typen (Cognis), insbesondere von gesättigten C₈₋₃₀-Fettsäuren,
- alkoxylierte Triglyceride,
- alkoxylierte Fettsäurealkylester der Formel (E4-1)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether,
- Fettsäurealkanolamide, Fettsäure-N-alkylglucamide und Fettamine sowie deren Ethylenoxid- oder Polyglycerin-Anlagerungsprodukte,
- Sorbitanfettsäureester und Anlagerungsprodukte von Ethylenoxid an Sorbitanfettsäureester, wie beispielsweise die Polysorbate,
- Zuckerfettsäureester und Methylglucosid-Fettsäureester sowie deren Ethylenoxid- oder Polyglycerin-Anlagerungsprodukte,
- Alkylpolyglycoside entsprechend der allgemeinen Formel RO-(Z)ₓ wobei R für einen Alkyl- oder Alkenylrest mit 4 bis 22 Kohlenstoffatomen, Z für einen Zuckerrest sowie x für die Anzahl der Zuckereinheiten steht. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die, wie oben beschrieben, erhalten werden können. Bevorzugt sind Alkyloligoglucoside auf Basis von gehärtetem C_{12/14}-Kokosalkohol mit einem DP von 1 bis 3. Besonders bevorzugt sind solche Alkylpolyglycoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.
Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.
- Gemische aus Alkyl-(oligo)-glucosiden und Fettalkoholen, z. B. Montanov^{®}68,
- Sterine, z. B. Ergosterin, Stigmasterin, Sitosterin und Mykosterine,
- Phospholipide, z. B. Lecithine bzw. Phosphatidylcholine,
- Polyglycerine und Polyglycerinderivate wie beispielsweise Polyglycerinpoly-12-hydroxystearat (Dehymuls^{®} PGPH) oder Triglycerindiisostearat (Lameform^{®} TGI),
- alkoxylierte Polydialkylsiloxane (INCI-Bezeichnung: Dimethicone Copolyol).
- Zuckertenside vom Typ der Fettsäure-N-alkylpolyhydroxyalkylamide, ein nichtionisches Tensid der Formel (E4-III),

   R⁵CO-NR⁶-[Z] (E4-III)

   in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁸-CH₂-(CHOH)₄-CH₂OH (E4-IV)

   Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmitoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.
Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.
Weiterhin sind ganz besonders bevorzugte nichtionische Tenside die Zuckertenside. Diese können in den erfindungsgemäß applizierten Reinigungszusammensetzungen bevorzugt in Mengen von 0,1 - 20 Gew.-%, besonders bevorzugt 0,5-15 Gew.-% und außerordentlich bevorzugt 1 - 7,5 Gew.%, jeweils bezogen auf die gesamte Zusammensetzung, enthalten sein.
Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.
Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Als bevorzugte nichtionische oberflächenaktive Substanzen haben sich die Alkylpolyglycoside, gegebenenfalls im Gemisch mit Fettalkoholen, alkoxylierte Polydialkylsiloxane, Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCl-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Bevorzugt einsetzbar sind erfindungsgemäß QAV mit Behenylresten, insbesondere die unter der Bezeichnung Behentrimoniumchlorid bzw. -bromid (Docosanyltrimethylammonium Chlorid bzw. - Bromid) bekannten Substanzen. Andere bevorzugte QAV weisen mindestens zwei Behenylreste auf, wobei QAV, welche zwei Behenylreste an einem Imidazoliniumrückgrat besonders bevorzugt sind. Kommerziell erhältlich sind diese Substanzen beispielsweise unter den Bezeichnungen Genamin^{®} KDMP (Clariant) und Crodazosoft^{®} DBQ (Croda).

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare® vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäß applizierten Reinigungszusammensetzungen bevorzugt in Mengen von 0,05 bis 10 Gew.-%, besonders bevorzugt 0,1 bis 5 Gew.-% und außerordentlich bevorzugt 0,5 - 2 Gew.-%, jeweils bezogen auf die gesamte Zusammensetzung, enthalten.

Die Tenside (E) werden bevorzugt in Mengen von 0,1 - 45 Gew.%, besonders bevorzugt 0,5 - 30 Gew.-% und außerordentlich bevorzugt 0,5 - 25 Gew.-%, jeweils bezogen auf die gesamte erfindungsgemäß applizierte Reinigungszusammensetzung, eingesetzt.

Anionische, nichtionische, zwitterionische und/oder amphotere Tenside sowie deren Mischungen können erfindungsgemäß bevorzugt sein.

In einer weiteren bevorzugten Ausführungsform können die erfindungsgemäß applizierten Reinigungszusammensetzungen Emulgatoren enthalten. Emulgatoren bewirken an der Phasengrenzfläche die Ausbildung von wasser- bzw. ölstabilen Adsorptionsschichten, die die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß bevorzugte Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®} 68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Bevorzugte erfindungsgemäße Zusammensetzungen sind dadurch gekennzeichnet, dass sie mindestens einen Emulgator in einer Gesamtmenge von 0,1 - 25 Gew.-%, bevorzugt 0,5-15 Gew.%, besonders bevorzugt 1-10 Gew.-% und außerordentlich bevorzugt 1,5 - 5 Gew.-%, jeweils bezogen auf die gesamte Reinigungszusammensetzung, enthalten.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 -15 können erfindungsgemäß besonders bevorzugt sein.

### Antimikrobielle Wirkstoffe

Da die erfindungsgemäßen Mittel insbesondere auch zur Anwendung im sehr empfindlichen Augenbereich vorgesehen sind, bedürfen sie einer wirksamen Konservierung durch antimikrobielle Wirkstoffe, insbesondere, wenn die Reinigungszusammensetzung Wasser enthält.
Erfindungsgemäß bevorzugte Konservierungsmittel sind Formaldehydabspalter (wie z. B. 1,3-Dimethylol-4,4-dimethylhydantoin, INCI-Bezeichnung DMDM Hydantoin, z. B. unter der Handelsbezeichnung Glydant von der Firma Lonza erhältlich), Iodopropylbutylcarbamate wie 3-lod-2-propinylbutylcarbamat (z. B. die unter den Handelsbezeichnungen Glycacil-L, Glycacil-S von der Firma Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), 2-Ethylhexylglycerin (Octoxyglycerin), Phenoxyethanol, Ethanol, Benzoesäure, Dibromdicyanobutan (2-Brom-2-brommethyl-glutarodinitril), 2-Brom-2-nitro-propan-1,3-diol, Imidazolidinylharnstoff, 5-Chlor-2-methyl-4-isothiazolin-3-on, 2- Chloracetamid, Benzalkoniumchlorid, Benzylalkohol, Salicylsäure und Salicylate, sowie Kombinationen dieser Rohstoffe.
Erfindungsgemäß besonders bevorzugte Konservierungsmittel sind ausgewählt aus lodopropylbutylcarbamaten, Parabenen (Methyl-, Ethyl-, Propyl- und/oder Butylparaben), 2-Ethylhexylglycerin und/oder Phenoxyethanol, sowie Kombinationen dieser Rohstoffe. Besonders bevorzugt ist 2-Ethylhexylglycerin.

Als antimikrobielle Komponente eignen sich weiterhin z. B. Phenole, Resorcine, Bisphenole, Salicylanilide und -amide sowie deren halogenierte Derivate, halogenierte Carbanilide und p-Hydroxybenzoesäureester. Beispielsweise sind halogenierte Diphenylether, wie 2,4-Dichlor-2'-hydroxydiphenylether, 4,4'-Dichlor-2'-hydroxydiphenylether, 2,4,4'-Tribrom-2'-hydroxydiphenylether, 2,4,4'-Trichlor-2'-hydroxydiphenylether (Triclosan) als antimikrobielle Wirkstoffe geeignet. Neben Bromchlorophen, Bisbiguaniden wie Chlorhexidin und Alexidin, Phenylsalicylsäureestern und 5-Amino-1,3-bis(2-ethylhexyl)-hexahydro-5-methylpyrimidin (Hexetidin) wirken auch Zink- und Kupferionen antimikrobiell, wobei synergistische Effekte insbesondere in Kombination mit Hexetidin und Triclosan auftreten. Auch quartäre Ammoniumverbindungen, wie z. B. Cetylpyridiniumchlorid, Benzalkoniumchlorid, Domiphenbromid und Dequaliniumchlorid sind einsetzbar. Als antimikrobiell wirksam haben sich auch Octapinol, Octenidine und Sanguinarin erwiesen. Die antimikrobiellen Wirkstoffe bzw. Konservierungsmittel sind in Mengen von 0,01 - 2, bevorzugt 0,1 - 1,5 und besonders bevorzugt 0,2 - 1,0 Gew.% enthalten, jeweils bezogen auf das Gewicht der erfindungsgemäß applizierten Reinigungszusammensetzung, enthalten.

Die erfindungsgemäß applizierten Reinigungszusammensetzungen können außerdem in bekannter Weise die üblicherweise auf dem betreffenden Gebiet verwendeten Zusatzstoffe enthalten, beispielsweise Antioxidantien, wie Vitamin E oder seine Derivate, Parfums, Füllstoffe, wie z.B. Kaolin oder Stärke, oder auch Mikrohohlkugeln, Pigmente, UV-Filter, Maskierungsmittel, etherische Öle, Farbmittel, hydrophile Wirkstoffe oder lipophile Wirkstoffe, entzündungshemmende und/oder hautberuhigende und/oder reizlindernde Wirkstoffe wie Allantoin, Bisabolol, Kornblumenwasser, Irisextrakt, Depigmentierungsmittel, feuchtigekeitsspendende Wirkstoffe, beispielsweise Harnstoff, Aminosäuren, Vitamine und ihre Derivate, Proteine, Salicylsäure und ihre Derivate, α-Hydroxysäuren, Pyrrolidoncarbonsäure und ihre Salze, Ceramide usw.

Der Fachmann wird selbstverständlich die gegebenenfalls vorliegende(n) Verbindungen) und/oder ihre Mengenanteile so auswählen, dass die vorteilhaften Eigenschaften der erfindungsgemäßen Zusammensetzung durch den beabsichtigten Zusatzstoff nicht oder nicht wesentlich beeinträchtigt werden.
Die Reinigungszusammensetzung weist einen pH-Wert auf, der für die Haut nicht nachteilig ist und der bevorzugt bei 5 bis 8 und besonders bevorzugt im Bereich von 5,5 bis 7,5 liegt.

Ein weiterer Gegenstand der Erfindung ist somit auch ein Mittel zur kosmetischen Reinigung, insbesondere zum Abschminken, umfassend einen stäbchenförmigen Einweg-Applikator, dessen Applikatorkopf mit einer Zusammensetzung zur Reinigung, insbesondere zum Abschminken, beaufschlagt ist und das einzeln verpackt ist.

### Applikatoren

Erfindungsgemäß bevorzugte Applikatoren zeichnen sich durch einen mehr oder weniger elastischen Applikatorkopf aus, mit dem der Anwender die zu reinigenden Haut- oder Schleimhautpartien behandeln kann. Um eine besonders gezielte Auftragung zu ermöglichen, ist es besonders vorteilhaft, wenn der Applikatorkopf eine möglichst kleine Auftragfläche aufweist. Vorteilhafterweise sind die Breite und die Länge der Auftragfläche jeweils kleiner als 0,5 cm, bevorzugt jeweils kleiner als 0,3 cm.
In einer besonders bevorzugten Ausführungsform ist die Form des Applikatorkopfes dem Profil der zu behandelnden Oberfläche angepasst, insbesondere durch eine angeschrägte Form des Applikatorkopfes.
Erfindungsgemäß besonders bevorzugt sind Tupfer- oder "Swab"-Applikatoren. Diese Art von Applikatoren sind vergleichbar mit den bekannten Wattestäbchen, deren Kopf mit der Reinigungszusammensetzung beaufschlagt ist, wobei der Anwender die zu reinigenden Haut- oder Schleimhautpartien mit dem getränkten Applikatorkopf bestreicht. Prinzipiell ist diese Art von Applikatoren auch in Verbindung mit pharmazeutischen und kosmetischen Präparaten möglich. Diese Einmal-Applikatoren sind in verschiedener Ausführungsform einsetzbar und bieten in ihrem Anwendungsbereich besondere Vorteile in Verbindung mit der beaufschlagten Zusammensetzung.

Generell liegt der Vorteil der Swab-Applikatoren zunächst in ihrem Einmal-Charakter, der dem Anwender ein Maximum an Hygiene garantiert. Ein weiterer Vorteil der Swab-Applikatoren liegt in der guten Handhabung, die eine gezielte Applikation der Zusammensetzung auf die Haut- oder Schleimhautoberfläche und damit eine besonders exakte Reinigung gewährleistet. Dies hat den Vorteil, das beispielsweise ein geringfügig übergezeichneter oder verschmierter Lidstrich oder eine Lippenkontur nur an der fehlerhaften Stelle gereinigt zu werden braucht, während der fehlerfrei geschminkte Teil des Make-ups erhalten bleiben kann. Weiterhin ist es von Vorteil, dass die Applikatoren in handlichen Verpackungseinheiten vertrieben werden können, die sich hervorragend für den Gebrauch unterwegs eignen. Weiterhin ist es von Vorteil, dass die Applikatoren nicht nur einzeln verpackt zu sein brauchen, sondern auch ohne eine für jeden einzelnen Applikator benötigte Verpackung zusammen in einer gemeinsamen Umverpackung auf den Markt gebracht werden können. Das gewährleistet einerseits eine kostengünstige Herstellung und andererseits eine bequeme Handhabung. Die Herstellung der Applikatoren ist vergleichsweise einfach und die Beaufschlagung oder Tränkung des Applikatorkopfes lässt sich exakt dosieren.

Wie schon dargelegt, gibt es unterschiedliche Ausführungsformen dieser Tupfer- oder Swab-Applikatoren, denen jedoch ein schlankes Röhrchen als Stiel und ein daran angebrachter Applikatorkopf gemeinsam sind. Es ist auch möglich, dass der Stiel an jedem Ende einen Applikatorkopf aufweist.

Zudem ist es vorteilhaft, dass die Applikatoren, insbesondere die Applikator-Köpfe besonders gut an die jeweiligen Erfordernisse angepasst werden kann. Dabei kann das Material des ApplikatorKopfes entsprechend der Viskosität so gewählt werden, dass die richtige Menge der Reinigungszusammensetzung haften bleibt. In einer bevorzugten Ausführungsform umfasst der Applikatorkopf Zellstofffasern. In einer anderen bevorzugten Ausführungsform ist der Applikatorkopf aus einem porösen Schaumstoff gefertigt, der die Reinigungszusammensetzung wie ein Schwamm aufnimmt. Der Vorteil des aus einem solchen Material geschnittenen Applikatorkopfes liegt auch darin, dass sich seine Form beliebig gestalten lässt. So können mehr oder weniger breite Applikationsflächen in beliebigem Anstellwinkel oder mit konkaver Krümmung vorgesehen werden. Mit derart in ihrer Form optimierten Applikator-Köpfen lassen sich auch Problemzonen gut erreichen.

Die folgenden Beispiele sollen den Gegenstand der Erfindung näher beschreiben (die Mengenangaben in der Beschreibung und in den Beispielen beziehen sich, sofern nicht anders angegeben, auf Gew.-%, bezogen auf das Gesamtgewicht der Reinigungszusammensetzung):

### Beispiel 1: Lotion zum Abschminken der Augen

| | |
|---|---|
| - FUCOGEL 1000 | 50 g |
| - 2-Ethylhexylpalmitat | 10 g |
| - Cyclopentadimethylsiloxan | 20 g |
| - Butylenglycol | 5 g |
| - Konservierungsmittel | qs |
| - Wasser | ad 100 g |

Diese Lotion, die keinen grenzflächenaktiven Stoff enthält, ermöglicht ein effizientes Abschminken, das für die Augen angenehm ist, auch bei wasserfester Schminke.

### Beispiel 2: Milch zum Abschminken

| | |
|---|---|
| - Octylpalmitat | 35 g |
| - Glycerin | 2 g |
| - FUCOGEL 1000 | 50 g |
| - Pemulen TR 1 | 0,1 g |
| -Triethanolamin | 0,1 g |
| - Weizenproteinhydrolysat | 1 |
| - Konservierungsmittel | qs |
| - Wasser | ad 100 g |

Diese Milch, die keinen grenzflächenaktiven Stoff enthält, weist gute kosmetische Eigenschaften auf.

| | Reinigungsmilch in Form einer O/W-Emulsion | Gesichtswasser | Duschbad | Waschgel, insbesondere für die Gesichtshaut |
|---|---|---|---|---|
| Dermofeel SL | 0,5 | - | - | - |
| Controx KS | 0,05 | - | 0,05 | 0,05 |
| Stenol 16/18 | 2,0 | - | - | - |
| Paraffinum Liquidum | 5,0 | - | - | - |
| Siliconöl 50 cSt | 3,0 | - | - | - |
| 2-Ethylhexylpalmitat | 3,0 | - | - | - |
| Methylparaben | 0,3 | - | - | - |
| Propylparaben | 0,1 | - | - | - |
| Amaze XT | 0,5 | 0,3 | 0,3 | 1,0 |
| Ethanol, 96 % | 10,0 | 10,0 | 10,0 | 5,0 oder 10,0 |
| d-Panthenol (75 %) | 5,0 | 1,0 | 0,5 | - |
| Parfum | 0,3 | 0,1 | 0,3 | 0,15 |
| 1,2-Propandiol | - | 7,0 | - | - |
| Cetiol HE | - | 0,5 | 0,5 | 0,5 |
| Acnacidol PG | - | 1,0 | - | - |
| Glycerin, 86 % | - | - | 6,0 | 6,0 |
| Sorbit, 70 % | - | - | - | 6,0 |
| Plantacare 1200 UP | - | - | 4,0 | - |
| Plantacare 2000 UP | - | - | 4,0 | 11,0 |
| Benzophenon-4 | - | - | 0,05 | 0,05 |
| Natriumbenzoat | - | - | 0,4 | 0,4 |
| Texapon NSO | - | - | - | 23,0 |
| Kamillenextrakt | - | - | - | 1,0 |
| Pfefferminzextrakt | - | - | - | 1,0 |
| Milchsäure, 80 % | - | - | - | 0,7 |
| Trilon B (flüssig) | - | - | 0,25 | 0,25 |
| Farbstoff E 131 | - | - | - | 0,0005 |
| Wasser | ad 100 | ad 100 | ad 100 | ad 100 |

### Abschminkzusammensetzungen in Form von dünnflüssigen Wasser-in-Öl-Emulsionen (< 5000 mPas)

| | Gew.-% |
|---|---|
| PEG-30-Dipolyhydroxystearat | 2,00 |
| Isohexadecan | 4,50 |
| Paraffinum liquidum | 12,50 |
| Glycerin | 3 00 |
| Magnesiumsulfat | 0,70 |
| Parfum, Konservierungsmittel, Farbstoffe, Antioxidantien | q.s. |
| Wasser | ad 100,00 |
| | Gew.-% |
| PEG-30-Dipolyhydroxystearat | 2,00 |
| Squalan | 6 00 |
| Paraffinum liquidum | 6,00 |
| Hydriertes Polysiobuten | 6,00 |
| Glycerin | 3,00 |
| Magnesiumsulfat | 0,70 |
| Parfum, Konservierungsmittel, Farbstoffe, Antioxidantien | q.s. |
| Wasser | ad 100,00 |
| | Gew.-% |
| PEG-30-Dipolyhydroxystearat | 2,00 |
| Cyloparaffin | 10,00 |
| Paraffinum liquidum | 7,50 |
| Tocopherolacetat | 0 50 |
| Glycerin | 3 00 |
| Panthenol | 0,30 |
| 1,3-Butylenglycol | 1,00 |
| Serin | 0,30 |
| Biotin | 0,10 |
| Distärkephosphat | 1 00 |
| Magnesiumsulfat | 0,70 |
| Parfum, Konservierungsmittel, Farbstoffe, Antioxidantien | q.s. |
| Wasser | ad 100,00 |

### Liste der verwendeten Rohstoffe:

| Handelsname | INCl-Bezeichnung/ Beschreibung | Hersteller |
|---|---|---|
| Amaze XT | Dehydroxanthan Gum | National Starch |
| Dermofeel SL | Natriumstearoyllactoyllactylat | Dr. Straetmans |
| Controx KS | Tocopherol, Hydrogenated Palm Glycerides Citrate | Cognis |
| Stenol 16/18 | Cetearylalkohol | Cognis |
| Cetiol HE | PEG-7 Glyceryl Cocoate | Cognis |
| Acnacidol PG | Propylene Glycol, 10-Hydroxydecanoic Acid, Sebacic Acid, 1,10-Decanediol | Vincience |
| Plantacare 1200 UP | Lauryl Glucoside, 50 - 53 % | Cognis |
| Plantacare 2000 UP | Decyl Glucoside, 51 - 55 % | Cognis |
| Texapon NSO | Laurylethersulfat-Natrium-Salz, 27 % in Wasser | Cognis |
| Trilon B (flüssig) | Na4-EDTA, 25 % in Wasser | BASF |
| Fucogel 1000 | Biosaccharide Gum-1 | Solabia |

## Patentansprüche

1. Mittel zur kosmetischen Reinigung, insbesondere zum Abschminken, umfassend einen stäbchenförmigen Einweg-Applikator, dessen Applikatorkopf mit einer Zusammensetzung zur Reinigung, insbesondere zum Abschminken, beaufschlagt ist.

2. Mittel nach Anspruch 1, **dadurch gekennzeichnet, dass** es einzeln verpackt ist.

3. Mittel nach einem der Ansprüche 1 oder 2, **dadurch gekennzeichnet, dass** die Reinigungszusammensetzung in Form einer flüssigen oder fließfähigen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, in wasserfreier Form, wie beispielsweise einem Öl oder einem Balsam, vorliegt.

4. Mittel nach einem der vorstehenden Ansprüche, **dadurch gekennzeichnet, dass** der Applikatorkopf Zellstofffasern umfasst.

5. Mittel nach einem der Ansprüche 1 - 3, **dadurch gekennzeichnet, dass** der Applikatorkopf einen porösen Schaumstoff umfasst.

6. Mittel nach einem der vorstehenden Ansprüche, **dadurch gekennzeichnet, dass** der Applikatorkopf eine Auftragfläche aufweist, deren Abmessungen jeweils kleiner als 0,5 cm sind.

7. Verwendung eines Mittels gemäß einem der Ansprüche 1 - 6 zur Reinigung und/oder zum Abschminken der Haut und/oder der Schleimhaut.

8. Nicht-therapeutisches Verfahren zur Reinigung und/oder zum Abschminken der Haut und/oder der Schleimhaut, wobei die zu reinigende Hautpartie mit einem stäbchenförmigen Einweg-Applikator, dessen Applikatorkopf mit einer Zusammensetzung zur Reinigung, insbesondere zum Abschminken, beaufschlagt ist, gereinigt wird.

9. Verfahren nach Anspruch 8, **dadurch gekennzeichnet, dass** der Einweg-Applikator einzeln verpackt ist.

10. Verfahren nach Anspruch 8 oder 9, **dadurch gekennzeichnet, dass** die Reinigungszusammensetzung in Form einer flüssigen oder fließfähigen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, in wasserfreier Form, wie beispielsweise einem Öl oder einem Balsam, vorliegt.

11. Verfahren nach einem der Ansprüche 8-10, **dadurch gekennzeichnet, dass** der Applikatorkopf Zellstofffasern umfasst.

12. Verfahren nach einem der Ansprüche 8 - 10, **dadurch gekennzeichnet, dass** der Applikatorkopf der Applikatorkopf einen porösen Schaumstoff umfasst.

13. Verfahren nach einem der Ansprüche 8 - 12, **dadurch gekennzeichnet, dass** der Applikatorkopf eine Auftragfläche aufweist, deren Abmessungen jeweils kleiner als 0,5 cm sind.
